# EUROPEAN PATENT APPLICATION

(11) **EP 4 599 769 A1**
(43) Date of publication of application: **13.08.2025**
(21) Application number: 24156735.3
(22) Date of filing: 09.02.2024
(51) Int. Cl.: A61B 6/00, A61B 6/58

(54) **ALIGNING IN AN X-RAY IMAGING SYSTEM**

(71) Applicant: Koninklijke Philips N.V., 5656 AG Eindhoven (NL)
(72) Inventor: GAST, Tomas, Eindhoven (NL); DRIES, Johan Juliana, Eindhoven (NL); VAN DER WACHT, Rogier Sean, 5656AG Eindhoven (NL); ELENBAAS, Thijs, Eindhoven (NL)
(74) Representative: Philips Intellectual Property & Standards

(57) **Abstract**

The present invention relates to aligning an X-ray tube and an X-ray detector that are movable in an independent manner. In order to provide a facilitated way of alignment for X-ray imaging systems, an X-ray imaging system (100) for medical imaging is provided. The system comprises a movable first holding arrangement (102) with a movably mounted X-ray source (104), a movable second holding arrangement (106) with a movably mounted X-ray detector (108), a controller (110) and an aligner (112). The controller is configured to control the X-ray source to generate an X-ray beam. The controller is configured to control the movement of the first and the second holding arrangement; wherein the first movable holding arrangement and the second movable holding arrangement are movable independently from each other. The X-ray detector has a detector surface area (116) to detect radiation generated by the X-ray source. The aligner is configured: to receive image data from the X-ray detector based on radiation generated by the X-ray source; to identify a predetermined geometric form (117) in the image data; to determine a spatial relation of the identified geometric form and the detector surface area; to compute a relative misalignment of the X-ray source and the X-ray detector based on the spatial relation; and to provide the computed relative misalignment to the controller for adjustment purposes of at least one of the first and second holding arrangements.

## Description

### FIELD OF THE INVENTION

The present invention relates to aligning an X-ray tube and an X-ray detector that are movable in an independent manner. The present invention relates in particular to an X-ray imaging system for medical imaging and to a method for aligning an X-ray imaging system for medical imaging.

### BACKGROUND OF THE INVENTION

In medical X-ray imaging, C-arm structures as commonly used in minimally invasive procedures can be replaced by two robotic arms that carry X-ray source and X-ray detector. This may be called the C-less concept. Precise alignment of both is essential when replacing scan movements of the C-arm structures. When using a robotic arm in a high-accuracy environment, it is necessary to calibrate the robotic arm, and in particular its end-effector for one or more points of interest in space, or the system is even calibrated in the full extent of the spanned space. Inferring the location from the motor encoders in each joint alone without calibration can be impossible. In industrial robotic arms, alignment may be provided by indicating off perpendicular surfaces to create a "reference frame". When two robotic arms are used together, not only the alignment between the arms and the room needs to be known, also the alignment between both arms becomes important. This is a requirement for the arms to perform tasks together, such as aligning an X-ray source and X-ray detector, and to prevent robot-to-robot collisions. This robot-to-robot alignment is currently performed in different ways, e.g. both robots perform or check calibration using the same known surfaces by touching it or they perform or check calibration with respect to each other's end-effector by touching. However, it has been shown that such calibrating by touching of the robotic arms is sometimes not accurate enough in medical imaging, even though proper alignment between the arms is important for 2D and 3D X-ray image quality as well as dose control and patient safety.

### SUMMARY OF THE INVENTION

There may thus be a need for providing a facilitated way of improved alignment for X-ray imaging systems.

The object of the present invention is solved by the subject-matter of the independent claims; further embodiments are incorporated in the dependent claims. It should be noted that the following described aspects of the invention apply also for the X-ray imaging system for medical imaging and for the method for aligning an X-ray imaging system for medical imaging.

In a preferred embodiment, an X-ray imaging system for medical imaging is provided. The system comprises a movable first holding arrangement with a movably mounted X-ray source, a movable second holding arrangement with a movably mounted X-ray detector, a controller and an aligner. The controller is configured to control the X-ray source to generate an X-ray beam. The controller is also configured to control the movement of the first and the second holding arrangement. The first movable holding arrangement and the second movable holding arrangement are movable independent from each other. The X-ray detector has a detector surface area to detect radiation generated by the X-ray source. The aligner is configured: to receive image data from the X-ray detector based on radiation generated by the X-ray source, to identify a predetermined geometric form in the image data, to determine a spatial relation of the identified geometric form and the detector surface area, to compute a relative misalignment of the X-ray source and the X-ray detector based on the determined spatial relation, and to provide the computed relative misalignment to the controller for adjustment purposes of at least one of the first and second holding arrangements.

As an advantage, alignment accuracy of the C-less concept is improved. As an effect, the installation time is reduced and thus the installation cost is reduced. Further, accuracy during operation is improved based on a facilitated detection of misalignments that allow earlier readjusting and compensation measures.

This way of alignment also provides the advantage that it can be performed for example as final alignment at the end of the installation procedure. This addresses that the tolerances on the X-ray system, the robotic arms, the installation procedure, and the installation site potentially influence the overall alignment from the X-ray source to the X-ray detector. An alignment at the end of the installation procedure takes all these tolerances into account, for example even when the subject support is already installed. Alternatively or in addition to this, the alignment procedure may just as well be provided periodically during use, or even during each imaging procedure to compensate for e.g. vibrations that are unique for that particular run.

The alignment procedure may be provided during standard imaging operations in addition to existing calibrations such as a rotational scan with dodecahedron calibration, that calibrates the system in a dynamic situation and with respect to a fixed point on the table.

The "controller" relates to a device or component or unit that provides controlling functions of the operation of the imaging system. The controller can be provided as separate unit or as integrated into a data processing unit.

The "aligner" relates to a device or component or unit that provides alignment functions of the holding arrangements of the imaging system.

The "detector surface area" relates to the part of the surface of the detector that actively provides image data as a result from being radiated. A portion of the X-ray beam i.e. X-ray radiation hitting the detector within a boundary comprising the detector surface area will contribute to the imaging process, while X-ray radiation hitting the detector outside the boundary surface area will not provide any contribution to the imaging process.

For example, it is preferable that an X-ray beam cross-section at the detector is the same size or smaller than the active detector surface area. In this case, the outer contour of the X-ray beam as a predetermined geometric form lies entirely within the active detector surface area, so that the spatial relation of the identified geometric form and the detector surface area can be determined in an accurate manner.

According to an example, the first holding arrangement is a first robotic arm with at least two segments movably mounted providing a plurality of degrees of freedom, which first robotic arm is mounted to a first base on one end; the X-ray tube is mounted to the free other end of the first robotic arm. The second holding arrangement is a second robotic arm with at least two segments movably mounted providing a plurality of degrees of freedom, which second robotic arm is mounted to a second base on one end; the X-ray detector is mounted the to the free other end of the second robotic arm.

According to an example, a subject support is arranged between the X-ray source and the X-ray detector.

According to an example, based on the computed relative alignment, the controller is configured to adjust the relative position of the X-ray detector and the X-ray source by adjusting the movement of at least one of the first holding arrangement and the second holding arrangement.

According to an example, the system is configured to generate and detect the X-ray beam and to compute the misalignment for a plurality of X-ray imaging positions.

As an option, the system is configured to generate and detect the X-ray beam and to compute the misalignment along a planned movement trajectory.

According to an example, the system is configured to perform a set of pre-defined moves, to generate and detect X-ray radiation at a number of pre-defined spatial positions, to reconstruct the locations of the X-ray source and X-ray detector carrying robotic system based on the shape of the predefined moves, and to identify misalignments along the pre-defined moves.

According to an example, the predetermined geometric form is an outer shape of the X-ray beam.

According to an example, for an identification the predetermined geometric form in the image data, the aligner is configured to provide an edge detection in the image data to separate X-ray field borders and signal or noise on the detector, and to calculate a most likely misalignment in the relative position of both arms, given the extracted parameters.

According to an example, the aligner is configured to identify the predetermined geometric form during a pre-procedural radiation procedure.

According to an example, the aligner is configured to identify the predetermined geometric form during recording of subject X-ray image data.

According to an example, for an operational alignment, the controller is configured to provide X-ray image data acquired during a medical imaging procedure in at least imaging position. The aligner is also configured to identify the predetermined geometric form in the provided image data, to determine the spatial relation of the identified geometric form and the detector surface area for the at least imaging position, and to compute a relative misalignment of the X-ray source and the X-ray detector for the at least imaging position.

According to the present invention, also a method is provided for aligning an X-ray imaging system for medical imaging that comprises a movable first holding arrangement with a movably mounted X-ray source and a movable second holding arrangement with a movably mounted X-ray detector having a detector surface area. The first movable holding arrangement and the second movable holding arrangement are movable independently from each other. The method comprises the following steps:
- generating an X-ray beam by the X-ray source;
- detecting X-ray radiation of the X-ray beam on the detector surface area;
- providing image data by the X-ray detector based on the detected radiation;
- identifying a predetermined geometric form in the image data;
- determining a spatial relation of the identified geometric form and the detector surface area; and
- computing a relative misalignment of the X-ray source and the X-ray detector based on the determined spatial relation.

The relative misalignment of the X-ray source and the X-ray detector can then be subject of an active compensation for alignment.

According to an aspect, it is provided to align two robotic systems relative to each other without direct line of sight, using an X-ray imaging chain. This technique is implemented through the geometry of the so-called C-less X-ray imaging systems, where one robotic arm carries an X-ray source, while the other robotic arm carries an X-ray detector.

The provided solution is applicable whenever two robotic systems are used where one has an X-ray source and one has an X-ray detector and where alignment is needed. The provided solution is in particular useful for alignment so in cases where a free line of sight between X-ray source and X-ray detector is not achievable for imaging positions.

In an example, for the alignment procedure, robots move through space and X-ray is on during the procedure, even though no subject is yet provided.

The image-data based identification of misalignment allows to perform the robot-to-robot alignment with two off-the-shelf robotic arms with high enough precision for the C-less geometry. This is of advantage over, for example, indicating of surfaces for alignment, i.e. touching a fixed surface or point in space with an end-effector on the robot arm, as this can be subjective and thus error-prone. The presently provided procedure improves the alignment accuracy. Compared to indicating of surfaces, which is a time-consuming process, another advantage is the reduction of the installation time.

The proposed image data based alignment is also of advantage for geometry of C-less X-ray imaging situations where direct optical line-of-sight is usually restricted which does not allow the robotic arms to indicate each other. With the C-less system, robot-to-robot alignment with line-of-sight is not always possible for all points spanning the space. For example, the robot arms cannot touch each other or a calibration object, if the table is in-between. Additionally, there is limited overlap of the workspace that can be physically reached by each robot, while the relative locations still need to be known. The proposed alignment also makes it a lot easier, faster, and cheaper to perform a re-alignment procedure.

The problem of possible misalignment in C-less X-ray imaging is exacerbated by the fact that the robots are placed on long, linear rails, so that a small angular alignment error will result in in large end effector errors. The present alignment also takes this into account by being able to provide detection of misalignment along all image positions.

An additional problem addressed by the proposed alignment is that robots are connected to different surfaces, i.e. the floor and ceiling of the room, which are beyond the control of the imaging system manufacturer. There may even be misalignments that change from scan to scan, for example when, during a scan, a heavy medical device is moved on the floor above.

According to an aspect, a collimated X-ray beam from a first robot is provided that is small enough to fit on the detector on the second robot and the edges of the X-ray field on the detector of the second robot are tracked. This will be done as an initial calibration spanning the room. According to another aspect, this calibration is also provided during the imaging procedure, so that non-structural errors can also be removed or reduced.

According to an aspect, the X-ray imaging chain that is already present in the C-less concept is used for alignment and calibration: A set of motions is performed that spans the entire workspace of the two robots. The X-ray is recorded on the detector. As the robots move, the image of the edges of the collimated X-ray source on the detector shifts, deforms, and rotates. An algorithm is used to translate the detector misalignments to the transformation matrix from one robot to the other. For initial calibration of the robot arms, it may be the case that registration is off, to the extent that the beam at certain locations partially or fully misses the detector. To resolve this, an iterative procedure is provided, starting with locations where the robots are close together, and inter-robot distance is increased, while continuously adjusting to have the beam edges to fall on top of the detector. In addition, in an example, a validation run is performed.

In addition or alternatively to this, the calibration can be done during procedures itself: As the robots move, the image of the edges of the collimated X-ray source on the detector shifts, deforms, and rotates. An algorithm is used to translate the detector misalignments to the transformation matrix from one robot to the other. This misalignment is used to correct the relative C-arm position on-the-fly, or in cases where spatial coherence over time is important (rotational scans, overlays, subtraction, etc.), the misalignment can be taken into account in the algorithms.

In an option, it is provided to do some basic alignment between the robots, otherwise the X-ray may not even hit the detector. An algorithm detects the edges of the X-ray field on the detector, and the non-perfect square projection is translated into relative misalignments between source and detector.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of an X-ray imaging system for medical imaging in the context of further equipment in an operational room in a lateral side view.
Fig. 2 schematically shows an example for a motion and alignment scheme of the system of Fig. 1.
Fig. 3 shows another example of an X-ray imaging system for medical imaging where the movable holding arrangements are provided as robotic arms. The example is shown in the context of an operational room, for example in a hospital.
Fig. 4 shows basic steps of an example of a method for aligning an X-ray imaging system for medical imaging.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

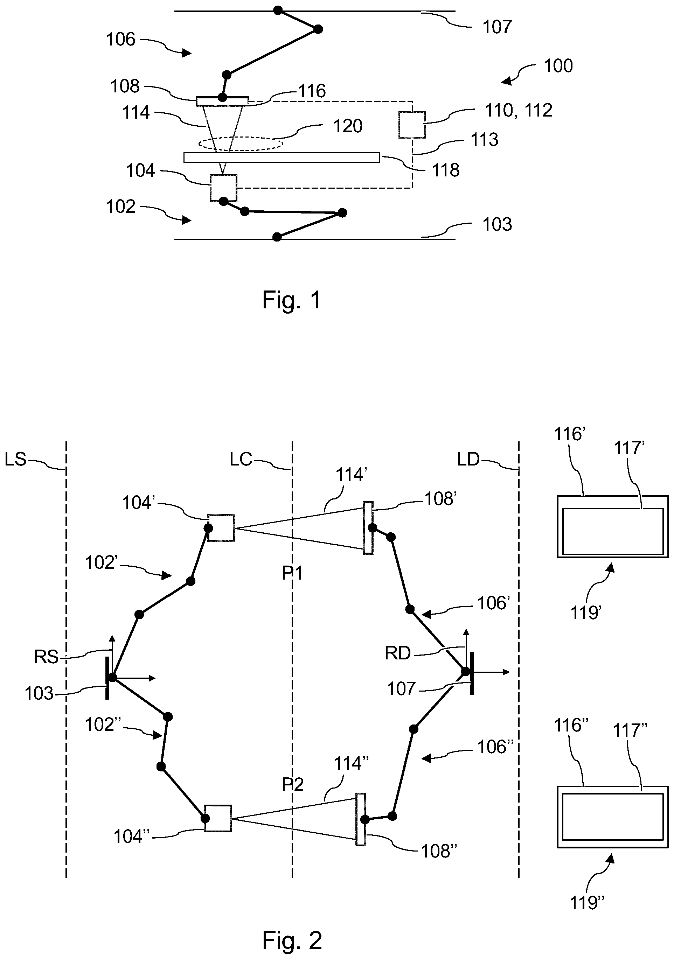

Fig. 1 schematically shows an example of an X-ray imaging system 100 for medical imaging. The system 100 comprises a movable first holding arrangement 102 with a movably mounted X-ray source 104, a movable second holding arrangement 106 with a movably mounted X-ray detector 108, a controller 110 and an aligner 112. The controller 110 is configured to control the X-ray source 104 to generate an X-ray beam 114 with a known spatial propagation (beam path) from the X-ray source 104 to the X-ray detector 108. The controller 110 is configured to control the movement of the first 102 and the second holding arrangement 106. The first movable holding arrangement 102 and the second movable holding arrangement 106 are movable independently from each other. The X-ray detector 108 has a detector surface area 116 to detect radiation generated by the X-ray source 104. The aligner 112 is configured to receive image data from the X-ray detector 108 based on radiation generated by the X-ray source 104, to identify a predetermined geometric form 117 (not shown in Fig. 1) in the image data, to determine a spatial relation of the identified geometric form and the detector surface area, to compute a relative misalignment of the X-ray source 104 and the X-ray detector 108 based on the determined spatial relation and to provide the computed relative misalignment to the controller 110 for adjustment purposes of at least one of the first and second holding arrangements.

In an example, the controller 110 and the aligner 112 are provided in an integrated manner. The controller 110 and the aligner 112 are data-connected to the X-ray tube 104 and the X-ray detector 108 by data-connections 113.

In an example, the motor encoders are also data-connected to the aligner.

As an option, the position of the robotic arm is provided in order to reconstruct the real position of the end-effector.

In an option, the aligner is provided as a software running on a data processer, on which other data processing may be provided as well.

In another option, the aligner is provided as a software within a designated data processor provided for this purpose.

As an option in Fig. 1, a subject support 118 is
arranged between the X-ray source 104 and the X-ray detector 108. A subject 120 to be imaged is also indicated in Fig. 1.

In an option, the movable first holding arrangement 102 is mounted to a floor 103, and the movable second holding arrangement 106 is mounted to a ceiling 107.

Instead of being mounted to the floor 103 or the ceiling 107, the movable first holding arrangement 102 and the movable second holding arrangement 106 can also be mounted to a wall structure or other parts of a building structure capable of providing sufficient support.

The "subject support" relates to a device for arranging the subject during imaging. An example for a subject support 118 is a subject table. The subject 120 can also be referred to as patient.

Fig. 2 schematically shows an example for a motion and alignment scheme of the system of Fig. 1 for two exemplary imaging states in a first position P1 and a second position P2. Even though the two positions are achievable only one after the other, they are shown in one drawing to indicate the range of possible imaging positions for which an alignment or mis-alignment is detected and, for example, actively corrected. The positions are indicated in an abstract manner. The X-ray viewing direction might be arranged in an upwards direction (e.g. the X-ray tube 104 below the subject support 118), a downwards direction (e.g. the X-ray tube 104 above the subject support 118), in a horizontal direction (e.g. the X-ray tube 104 lateral to the subject support 118) or in an oblique or angled manner.

The movable first holding arrangement 102 is indicated in the first position P1 with reference numeral 102' and in the second position P2 with reference numeral 102". The X-ray tube 104 is indicated with reference numerals 104' and 104" respectively. A first reference frame RS of the X-ray source 104 is indicated for the movable first holding arrangement 102.

The movable second holding arrangement 106 is indicated in the first position P1 with reference numeral 106' and in the second position P2 with reference numeral 106". The X-ray detector 108 is indicated with reference numerals 108' and 108" respectively. A second reference frame RD of the X-ray detector 108 is indicated for the movable second holding arrangement 106.

A range of motion for the X-ray source is indicated by a center line LC in the middle and a source line LS on the left of the center line LC; a range of motion for the X-ray detector is indicated by a center line LC and a detector line LD on the right of the center line LC. The first position P1 is indicated in the upper half of Fig. 2, the second position P2 in the lower half of Fig. 2.

In a right column of Fig. 2, illustrations are provided that indicate an image as detected by the detector 108 on its detector surface area 116. The image also shows a predetermined geometric form 117.

In the upper part, a first illustration 119' of the image is indicated for the first position P 1. In the lower part, a second illustration 119" of the image is indicated for the second position P2. As an example, a misalignment of the first position P1 can be detected based on the spatial relation between the predetermined geometric form 117, in this example an outer shape of the X-ray beam, and the detector surface area 116. In the first position P1, the spatial relation includes an offset of the predetermined geometric form 117 with respect to the detector surface area 116. In other words, the center of the predetermined geometric form 117 has shifted with respect to the center of the detector surface area 116.

To the contrary, in the second position P2, the predetermined geometric form 117 is centered within the detector surface area 116, their mutual spatial relation therefore indicating an aligned positioning. Of course, as an alignment, also other relative arrangements of the predetermined geometric form 117 and the detector surface area 116 can be envisaged. For example, in addition or as an alternative to the translation or shift of the geometric form 117 displayed here, a rotation of the geometric form 117 with respect to the detector area 116 may also be indicative of a misalignment between the robot arms. Accordingly, the spatial relation between the geometric form 117 and the detector area 116 may include a rotation angle of the geometric form 117 with respect to an outer edge of the detector surface area 116 other than 0 degrees, for example.

Hence, Fig. 2 shows a simplified view of the proposed robot-to-robot alignment, showing the positions of robotic arms on the left and the resulting X-ray detector image on the right. This image shows that the arms are not aligned since the X-ray image shifts when the arms move.

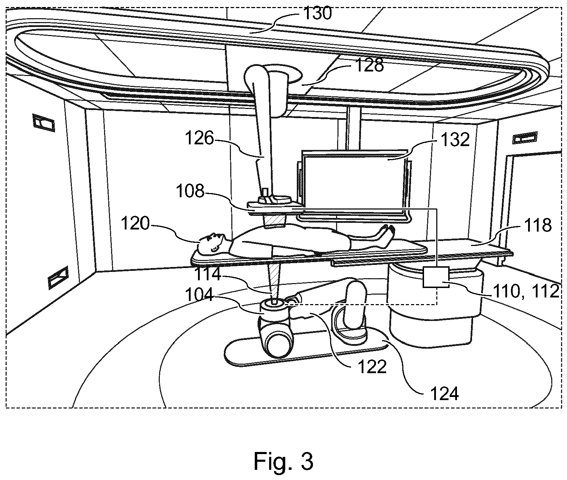

Fig. 3 shows another example of the X-ray imaging system 10 for medical imaging where the movable first and second holding arrangements 102, 106 are provided as robotic arms. The first holding arrangement 102 is a first robotic arm 122 with at least two segments movably mounted providing a plurality of degrees of freedom. The first robotic arm 122 is mounted to a first base 124 on one end, and the X-ray tube 104 is mounted to the free other end of the first robotic arm 122. The second holding arrangement 106 is a second robotic arm 126 with at least two segments movably mounted providing a plurality of degrees of freedom. The second robotic arm 126 is mounted to a second base 128 on one end. The X-ray detector 108 is mounted the to the free other end of the second robotic arm 126. For example, the second base 128 can be mounted to movable structure, like ceiling rails 130. Further, a display arrangement 132 is provided.

As an example, the first base 124 is mounted to the floor 103 or to a rail system on the floor 103. As another example, the second 128 is mounted to the ceiling 107 or to a rail system attached to the ceiling 107.

In an example, at least one of the first and second robot arms has six joints to achieve six degrees of freedom positioning in 3D space. In an option, seven joints are provided. In an example, both the first and the second robot arms have six joints each. In an option, both have seven joints.

In an option, not shown in detail, based on the computed relative alignment, the controller 110 is configured to adjust the relative position of the X-ray detector 108 and the X-ray source 104 by adjusting the movement of at least one of the first holding arrangement 102 and the second holding arrangement 106.

As an important effect, the misalignments can be compensated for in real-time, e.g. sagging, not just in software correction, but also physically, since the robots have enough degrees of freedom (axis) to make such small adjustments.

In an option, the computed misalignments are used to predict maintenance. As an example, the alignment changes over time as the backlash in gears increases due to wear. Re-running alignment comes at the cost of downtime.

In an option, an alignment guard is provided that constantly records the image data on the detector. Whenever the arms reach a certain position which is part of the alignment procedure, the algorithm re-checks the alignment. If this shows a drift over time, re-alignment / maintenance is required.

In an option, not shown in detail, the system is configured to generate and detect the X-ray beam and to compute the misalignment for a plurality of X-ray imaging positions. In an example, the system is configured to generate and detect the X-ray beam and to compute the misalignment along a planned movement trajectory.

In an option, not shown in detail, the system is configured: to perform a set of pre-defined moves, to generate and detect X-ray radiation at a number of pre-defined spatial positions, to reconstruct the locations of the X-ray source and X-ray detector carrying robotic system based on the shape of the predefined moves, and to identify misalignments along the pre-defined moves.

In an option, not shown in detail, the predetermined geometric form is an outer shape of the X-ray beam.

In an option, not shown in detail, for an identification the predetermined geometric form in the image data, the aligner 112 is configured: to provide an edge detection in the image data to separate X-ray field borders and signal or noise on the detector, and to calculate a most likely misalignment in the relative position of both arms, given the extracted parameters.

In a simple example, the misalignment is a translation vector plus rotation vector adjustment. In another example, the misalignment is more complex, e.g. concave linear axis, bending arms under their weight or the like, while it still aimed to solve this using the simple misalignment vectors. The overall correction would then be the one that results in the 'overall best performing misalignment correction', even though this will knowingly result in a misalignment.

In an example, the calculating of the most likely misalignment is provided by a geometrical simulator.

In an example, each corner point yields two parameters, where for the full deformed square fields, eight parameters are fitting on the edges allowing for sub-pixel resolution of the parameters.

In an option, it is provided to temporally average the shift, to further reduce noise and achieve subpixel resolution.

In an option, not shown in detail, for the image data generation, to measure a shift over time, the system is configured to i) move to fixed locations, stopping, taking an image, and then moving to the next point; or ii) the images are taken constantly during the moves creating a video, either pre-procedurally or while recording patient images.

In an example, a system is provided that makes corrections between moves.

In another example, a system is provided that collects all images first and then makes a correction at the end.

In an option, not shown in detail, the aligner 112 is configured to identify the predetermined geometric form during a pre-procedural radiation procedure.

In an option, not shown in detail, the aligner 112 is configured to identify the predetermined geometric form during recording of subject X-ray image data.

In an option, not shown in detail, for an initial alignment, the controller 110 is configured to: arrange the X-ray source and the X-ray detector in a starting position, in which the X-ray source and the X-ray detector are facing each other, to generate an initial X-ray beam and detecting X-ray radiation by the X-ray detector; and the aligner 112 is configured to identify if complete cross-section of X-ray beam is detected by the X-ray detector; and the controller 110 is configured: to adjust a distance between the X-ray source and the X-ray detector to provide X-ray beam on maximum surface area of the X-ray detector, and to generate a further X-ray beam and detecting the X-ray radiation by the X-ray detector as well as to identify if complete cross-section of X-ray beam is detected by the X-ray detector, and to further adjust the X-ray source and the X-ray detector to provide optimum alignment during imaging.

The "initial alignment" relates to an alignment procedure that takes place upon installation of the system.

In an example, the maximum surface is the area that is thought to have enough margin to be safe during the motion.

In an option, the beam is collimated or the detector to tube distance is reduced to reduce the beam's area that is hitting the detector.

If necessary, the loop is repeated.

In an option, not shown in detail, for an operational alignment, the controller 110 is configured to provide X-ray image data acquired during a medical imaging procedure in at least imaging position; and the aligner 112 is configured: to identify the predetermined geometric form in the provided image data, to determine the spatial relation of the identified geometric form and the detector surface area for the at least imaging position, and to compute a relative misalignment of the X-ray source and the X-ray detector for the at least imaging position.

The "operational alignment" relates to an alignment procedure that takes place during the normal operation of the system.

In an example, collimation of the X-ray beam is provided.

A first application of the alignment procedure based on image data is to correct misalignments upon installations.

A second application of the alignment procedure based on image data is to correct misalignments during use. A first option is to collect data and to provide the correction of misalignments in certain intervals, e.g. on a daily, weekly or monthly or even longer basis, or on a general maintenance routine basis. A second option is to collect data and to provide the correction of misalignments during the actual imaging procedure by actively changing the tube's and/or detector's movement, i.e. its position.

The detection and the correction may take place as two separate moments in time.

In an option, the misalignment is detected, and an alert is provided. Upon the alert, as a first option, calibration in terms of software-based correction is provided. As a second option, collimation is provided. As a third option, re-alignment is provided.

In order to facilitate edge detection, sharp beam edges are provided. For example, tube-side collimation is provided.

For calibration upon setup, a largest beam as possible is generated.

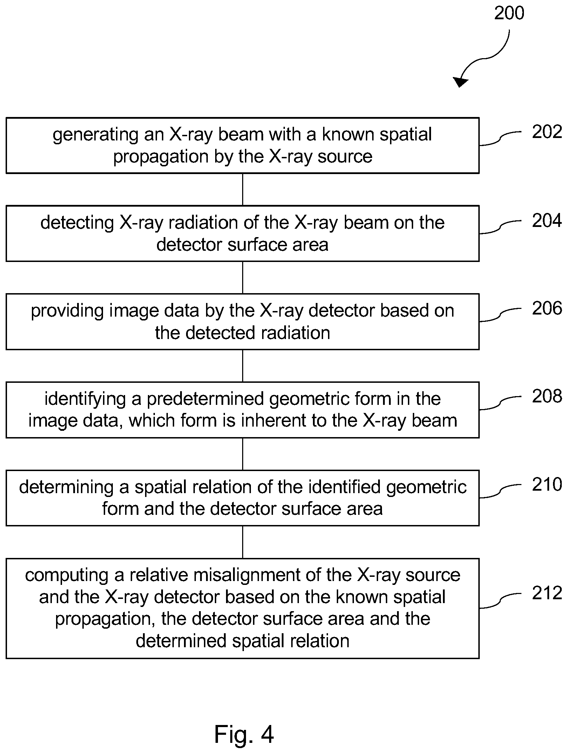

Fig. 4 shows basic steps of an example of a method 200 for aligning an X-ray imaging system for medical imaging, that comprises a movable first holding arrangement with a movably mounted X-ray source and a movable second holding arrangement with a movably mounted X-ray detector having a detector surface area; the first movable holding arrangement and the second movable holding arrangement are movable independently from each other. The method comprises the following steps:
- In a first step 202, an X-ray beam is generated by the X-ray source. Generally, the X-ray beam will have a known spatial propagation from the X-ray source towards the X-ray detector, in operation passing through an object to be examined.
- In a second step 204, X-ray radiation of the X-ray beam on the detector surface area is detected.
- In a third step 206, image data by the X-ray detector is provided based on the detected radiation.
- In a fourth step 208, a predetermined geometric form in the image data is identified. Preferably, this geometric form is inherent to the X-ray beam, such as an outer contour or shape of the X-ray beam. This form may, for example, be determined by a beam limiting device that is provided adjacent the X-ray source, such as a collimator.

In another example, the predetermined geometric form may be that of an absorbing device being arranged within the X-ray beam path.
- In a fifth step 210, a spatial relation of the identified geometric form and the detector surface area is determined.
- In a sixth step 212, a relative misalignment of the X-ray source and the X-ray detector is computed based on the determined spatial relation, preferably also taking into consideration the known spatial propagation of the X-ray beam from the X-ray source to the X-ray detector and geometric properties of the detector surface area.

In an example of the method, based on the computed relative alignment, it is further provided the step of
- adjusting the relative position of the X-ray detector and the X-ray source by adjusting the movement of at least one of the first holding arrangement and the second holding arrangement.

In an example of the method, the X-ray beam is generated and detected, and the misalignment is computed for a plurality of X-ray imaging positions. In an option, the X-ray beam is generated and detected, and the misalignment is computed along a planned movement trajectory.

In an example of the method, it is provided:
- performing a set of pre-defined moves;
- generating and detecting X-ray radiation at a number of pre-defined spatial positions;
- reconstructing the locations of the X-ray source and X-ray detector carrying robotic system based on the shape of the predefined moves; and
- identifying the 'X-ray beam center relative to the detector center.

For example, for identifying it is provided the step of identifying misalignments along the pre-defined moves.

In an example of the method, the predetermined geometric form is an outer shape of the X-ray beam.

In an example of the method, for identifying the predetermined geometric form in the image data, it is provided the steps of:
- edge detecting in the image data to separate X-ray field borders and signal or noise on the detector; and
- calculating a most likely misalignment in the relative position of both arms, given the extracted parameters.

In an example of the method, for the image data generation, to measure a shift over time it is provided one of the following:
i) the system is moving to fixed locations, is stopping, taking an image, and is then moving to the next point; or
ii) the images are taken constantly during the moves creating a video, either pre-procedurally or while recording patient images.

In an example of the method, the identifying of the predetermined geometric form is provided during a pre-procedural radiation procedure.

In an example of the method, the identifying of the predetermined geometric form is provided during recording of subject X-ray image data.

In an example of the method, for an initial alignment, it is provided the steps of:
- arranging the X-ray source and the X-ray detector in a starting position, in which the X-ray source and the X-ray detector are facing each other;
- generating an initial X-ray beam and detecting X-ray radiation by the X-ray detector;
- identifying if complete cross-section of X-ray beam is detected by the X-ray detector;
- adjusting distance between the X-ray source and the X-ray detector to provide X-ray beam on maximum surface area of the X-ray detector;
- generating a further X-ray beam and detecting the X-ray radiation by the X-ray detector as well as identifying if complete cross-section of X-ray beam is detected by the X-ray detector; and
- further adjusting of the X-ray source and the X-ray detector to provide optimum alignment during imaging.

In an example of the method, for an operational alignment, it is provided the steps of:
- providing X-ray image data acquired during a medical imaging procedure in at least imaging position;
- identifying the predetermined geometric form in the provided image data;
- determining the spatial relation of the identified geometric form and the detector surface area for the at least imaging position; and
- computing a relative misalignment of the X-ray source and the X-ray detector for the at least imaging position.

In an example, a computer program is provided comprising instructions which, when the program is executed by a computer, cause the computer to carry out the method of one of the previous examples.

In an example, a computer program or program element for controlling an apparatus according to one of the examples above is provided, which program or program element, when being executed by a processing unit, is adapted to perform the method steps of one of the method examples above.

In another example, a computer readable medium is provided having stored the computer program of the preceding example.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray imaging system (100) for medical imaging; the system comprising:
- a movable first holding arrangement (102) with a movably mounted X-ray source (104);
- a movable second holding arrangement (106) with a movably mounted X-ray detector (108);
- a controller (110); and
- an aligner (112);
wherein the controller is configured to control the X-ray source to generate an X-ray beam;
wherein the controller is configured to control the movement of the first and the second holding arrangement; wherein the first movable holding arrangement and the second movable holding arrangement are movable independently from each other;
wherein the X-ray detector has a detector surface area (116) to detect radiation generated by the X-ray source; and
wherein the aligner is configured: to receive image data from the X-ray detector based on radiation generated by the X-ray source; to identify a predetermined geometric form (117) in the image data; to determine a spatial relation of the identified geometric form and the detector surface area; to compute a relative misalignment of the X-ray source and the X-ray detector based on the determined spatial relation; and to provide the computed relative misalignment to the controller for adjustment purposes of at least one of the first and second holding arrangements.

2. System according to claim 1, wherein the first holding arrangement is a first robotic arm (122) with at least two segments movably mounted providing a plurality of degrees of freedom, which first robotic arm is mounted to a first base on one end, wherein the X-ray tube is mounted to the free other end of the first robotic arm; and
wherein the second holding arrangement is a second robotic arm (126) with at least two segments movably mounted providing a plurality of degrees of freedom, which second robotic arm is mounted to a second base on one end, wherein the X-ray detector is mounted the to the free other end of the second robotic arm.

3. System according to claim 1 or 2, wherein a subject support (118) is arranged between the X-ray source and the X-ray detector.

4. System according to claim 1, 2 or 3, wherein, based on the computed relative alignment, the controller is configured to adjust the relative position of the X-ray detector and the X-ray source by adjusting the movement of at least one of the first holding arrangement and the second holding arrangement.

5. System according to one of the preceding claims, wherein the system is configured to generate and detect the X-ray beam and to compute the misalignment for a plurality of X-ray imaging positions; and
wherein the system is configured to generate and detect the X-ray beam and to compute the misalignment along a planned movement trajectory.

6. System according to one of the preceding claims, wherein the system is configured: to perform a set of pre-defined moves; to generate and detect X-ray radiation at a number of pre-defined spatial positions; to reconstruct the locations of the X-ray source and X-ray detector carrying robotic system based on the shape of the predefined moves; and to identify misalignments along the pre-defined moves.

7. System according to one of the preceding claims, wherein the predetermined geometric form is an outer shape of the X-ray beam.

8. System according to one of the preceding claims, wherein, for an identification the predetermined geometric form in the image data, the aligner is configured to provide an edge detection in the image data to separate X-ray field borders and signal or noise on the detector; and to calculate a most likely misalignment in the relative position of both arms, given the extracted parameters.

9. System according to one of the preceding claims, wherein, for the image data generation, to measure a shift over time the system is configured to one of the following:
i) the system moves to fixed locations, stops, takes an image, and is then moving to the next point; or
ii) the images are taken constantly during the moves creating a video, either pre-procedurally or while recording patient images.

10. System according to one of the preceding claims, wherein the aligner is configured to identify the predetermined geometric form during a pre-procedural radiation procedure.

11. System according to one of the preceding claims, wherein the aligner is configured to identify the predetermined geometric form during recording of subject X-ray image data.

12. System according to one of the preceding claims, wherein, for an initial alignment, the controller is configured to arrange the X-ray source and the X-ray detector in a starting position, in which the X-ray source and the X-ray detector are facing each other; to generate an initial X-ray beam and detecting X-ray radiation by the X-ray detector; and the aligner is configured to identify if complete cross-section of X-ray beam is detected by the X-ray detector; and the controller is configured to adjust a distance between the X-ray source and the X-ray detector to provide X-ray beam on maximum surface area of the X-ray detector; and to generate a further X-ray beam and detecting the X-ray radiation by the X-ray detector as well as to identify if complete cross-section of X-ray beam is detected by the X-ray detector; and to further adjust the X-ray source and the X-ray detector to provide optimum alignment during imaging.

13. System according to one of the preceding claims, wherein, for an operational alignment, the controller is configured to provide X-ray image data acquired during a medical imaging procedure in at least imaging position; and the aligner is configured to identify the predetermined geometric form in the provided image data; to determine the spatial relation of the identified geometric form and the detector surface area for the at least imaging position; and to compute a relative misalignment of the X-ray source and the X-ray detector for the at least imaging position.

14. A method (200) for aligning an X-ray imaging system for medical imaging comprising a movable first holding arrangement with a movably mounted X-ray source and a movable second holding arrangement with a movably mounted X-ray detector having a detector surface area, wherein the first movable holding arrangement and the second movable holding arrangement are movable independently from each other, the method comprising the following steps:
- generating (202) an X-ray beam by the X-ray source;
- detecting (204) X-ray radiation of the X-ray beam on the detector surface area;
- providing (206) image data by the X-ray detector based on the detected radiation;
- identifying (208) a predetermined geometric form in the image data;
- determining (210) a spatial relation of the identified geometric form and the detector surface area; and
- computing (212) a relative misalignment of the X-ray source and the X-ray detector based on the known spatial propagation.

15. Computer program comprising instructions which, when the program is executed by a computer, cause the computer to carry out the method of claim 14.
